# EUROPEAN PATENT SPECIFICATION

(11) **EP 3 604 374 B1**
(45) Date of publication and mention of the grant of the patent: **20.07.2022**
(21) Application number: 18774657.3
(22) Date of filing: 06.02.2018
(51) Int. Cl.: C08G 18/36, C07C 69/30, C08G 18/32, C08K 7/02, C08L 75/04

(54) **POLYOL COMPOSITION FOR POLYURETHANE RESIN, POLYURETHANE RESIN-FORMING COMPOSITION, AND COMPOSITE MATERIAL**
POLYOLZUSAMMENSETZUNG FÜR POLYURETHANHARZ, POLYURETHANHARZFORMENDE ZUSAMMENSETZUNG UND VERBUNDSTOFF
COMPOSITION DE POLYOLS POUR UNE RÉSINE DE POLYURÉTHANNE, COMPOSITION FORMANT UNE RÉSINE DE POLYURÉTHANNE, ET MATÉRIAU COMPOSITE

(30) Priority: 31.03.2017 JP 2017071071
(43) Date of publication of application: 05.02.2020
(73) Proprietor: Dai-Ichi Kogyo Seiyaku Co., Ltd., Kyoto-shi, Kyoto 600-8873 (JP)
(72) Inventor: OIKAWA, Kei, kyoto-shi Kyoto 600-8873 (JP); HIROSE, Masaharu, kyoto-shi Kyoto 600-8873 (JP); MIYAMURA, Takeshi, kyoto-shi Kyoto 600-8873 (JP)
(74) Representative: TBK
(86) International application number: PCT/JP2018/004096
(87) International publication number: WO 2018/179868

(56) References cited:
- GB-A- 1 516 340
- JP-A- H 101 526
- JP-A- 2005 023 162
- JP-A- 2005 023 162
- JP-A- 2005 263 240
- JP-A- 2006 077 121
- JP-A- 2006 077 121
- JP-A- 2017 002 265
- US-A1- 2009 318 657
- EL-GHAZAWY ET AL: "Surface and thermodynamic properties for some novel polyoxyalkylenated trimethylolpropane monoester surfactants", COLLOIDS AND SURFACES A: PHYSIOCHEMICAL AND ENGINEERING ASPECTS, ELSEVIER, AMSTERDAM, NL, vol. 260, no. 1-3, 15 June 2005 (2005-06-15), pages 1-6, XP027803244, ISSN: 0927-7757 [retrieved on 2005-06-15]

## Description

### Technical Field

The present invention relates to a polyol composition for a polyurethane resin, a polyurethane resin-forming composition, and a composite material. More specifically, the present invention relates to a polyol composition for a polyurethane resin and a polyurethane resin-forming composition that exhibit such a low resin viscosity during shaping that they are highly usable, and a composite material.

### Background Art

Fiber-reinforced composite materials (FRPs) are lightweight but have excellent properties. For this reason, FRPs are used in wide-ranging applications in electric and electronic components, vehicles, and aviation, for example. FRPs contain, as matrix resins, thermosetting resins such as epoxy resins. However, these thermosetting resins exhibit such a high resin viscosity during shaping that they exhibit poor fluidity. In particular, there is room for improvement in the usability during production of large-sized products.

In order to address this, a technique has been disclosed that employs, as matrix resin, a polyurethane resin to provide a lower resin viscosity during shaping to achieve improved usability (Patent Literature 1).

JP 2005-023162 A relates to a polyurethane resin comprising a polyol (A) represented by general formula (I): 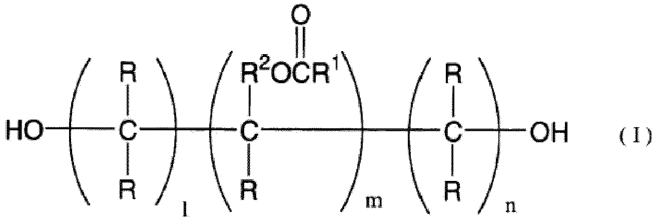 wherein R¹ and R² are each an aliphatic group, an alicyclic group or an aromatic group in which the number of carbon atoms of one or both of R¹ and R² is ≥6; Rs are each hydrogen, an aliphatic group, an alicyclic group or an aromatic group and may be different, respectively: n is an integer of 0-3; m is an integer of 1-3; and/or general formula (II): 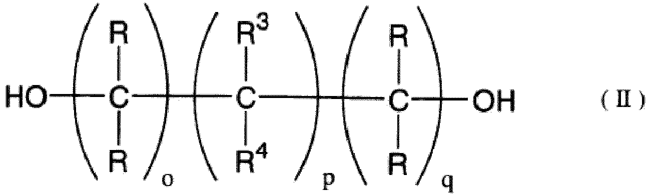 wherein R³s are each a ≥6C aliphatic group, alicyclic group or aromatic group; R⁴s are each hydrogen, an aliphatic group, an alicyclic group or an aromatic group and may be different, respectively; Rs are each hydrogen, an aliphatic group, an alicyclic group or an aromatic group and may be different, respectively: o and q are each an integer of 0-3; p is an integer of 1-3; and a polyisocyanate (B) as constituent component.

JP 2006-077121 A relates to a coating agent comprises a polyurethane resin comprising one or more diols (A) selected from a group consisting of a specific structural formula such as general formula (I): 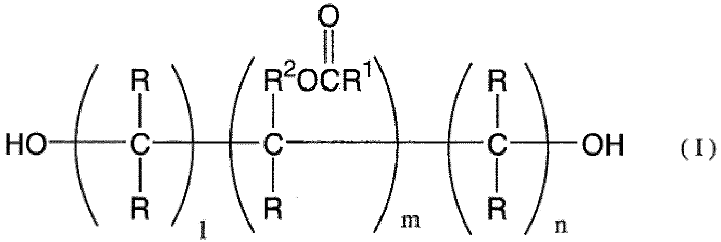 , dimer diols and hydrogenated dimer diols, a polyester polyol (B) prepared by copolymerizing an aromatic acid component with an alicyclic acid component, an aromatic polyisocyanate (C) and, if needed, a compound (D) having a side chain containing ≥2 functional groups reacting with isocyanate in one molecule and <1000 molecular weight, and carbon-based powder.

US 2009/318657 A1 relates to a polyisocyanate component having an isocyanate group content of 5 to 38% by weight and average 2 to 3 functional groups, obtained by modifying an aliphatic isocyanate and/or an alicyclic isocyanate, a polyol component having a hydroxyl value of 20 to 350 mgKOH/g and average 2 to 3 functional groups, and a glycerin fatty acid ester having hydroxyl group(s).

GB 1516340 A relates to a process for the production of anionic polyurethanes, wherein a monomeric, aliphatic dihydroxyl compound with an aliphatic substituent containing at least 10 carbon atoms, in which the aliphatic chain joining the two hydroxyl group and/or the aliphatic substituent may contain a hetero atom, provided that where a hetero atom is present in the chain it is not substituted by any radical which is reactive with isocyanate groups is reacted with a polyisocyanate to form a preadduct containing terminal NCO-groups, and the preadduct obtained is chain-extended either with an aliphatic, monomeric diol containing an acid group capable of salt formation and the acid group converted completely or in part into a salt by reaction with a base, or with an aliphatic monomeric diol which contains an acid group which has already been completely or partly neutralised to the corresponding salt group.

R.A. El-Ghazawy (Colloids and Surfaces A: Physicochem. Eng. Aspects, Volume 260, Issues 1-3, 2005, Pages 1-6) relates to three series of polyoxyethylenated trimethylolpropane (TMP) monoester surfactants.

### Citation List

### Patent Literature

PTL 1: Japanese Unexamined Patent Application Publication (Translation of PCT Application) No. 2014-506606

### Summary of Invention

### Technical Problem

However, the polyurethane resin described in PTL 1 has such a low glass transition temperature of about 60°C to about 70°C that it is used in limited applications.

In order to address such existing problems or the like, an object of the present invention is to provide a polyol composition for a polyurethane resin and a polyurethane resin-forming composition that are capable of forming a polyurethane resin that exhibits a low resin viscosity during shaping, but has a high glass transition temperature, and a composite material.

### Solution to Problem

In order to achieve the object, a polyol composition for a polyurethane resin, a polyurethane resin-forming composition, and a composite material according to the present invention mainly include the following features.

A polyol composition for a polyurethane resin, including an esterified compound (A) having an average esterification degree of 0.5 to 2.5, wherein the esterified compound (A) includes, as constituent components, a triol (a1) and a fatty acid (a2) having 4 to 12 carbon atoms and wherein the esterified compound (A) content of the polyol composition for a polyurethane resin is 80 mass% or more.

Because of this feature, the polyol composition for a polyurethane resin is capable of forming a polyurethane resin that exhibits a low resin viscosity during shaping, but has a high glass transition temperature.

### Advantageous Effects of Invention

The present invention provides a polyol composition for a polyurethane resin and a polyurethane resin-forming composition that are capable of forming a polyurethane resin that exhibits a low resin viscosity during shaping, but has a high glass transition temperature, and a composite material.

### Description of Embodiments

### <Polyol composition for polyurethane resin>

A polyol composition for a polyurethane resin according to an embodiment of the present invention includes an esterified compound (A) having an average esterification degree of 0.5 to 2.5. The esterified compound (A) includes, as constituent components, a triol (a1) and a fatty acid (a2) having 4 to 12 carbon atoms. Hereinafter, these will be individually described.

### (Esterified compound (A))

The esterified compound (A) includes, as constituent components, the triol (a1) and the fatty acid (a2) having 4 to 12 carbon atoms.

### Triol (a1)

The triol (a1) is not particularly limited. Examples of the triol (a1) include glycerin, trimethylolpropane, and triethanolamine. Such triols (a1) may be used in combination. In particular, the triol (a1) is, from the viewpoint of exhibiting a lower viscosity during shaping and providing a polyurethane resin having a higher glass transition temperature, preferably glycerin or trimethylolpropane, more preferably trimethylolpropane.

The triol (a1) content is not particularly limited. For example, the triol (a1) content of the polyol contained in the constituent components of the esterified compound (A) is, from the viewpoint of providing a polyurethane resin having a higher glass transition temperature, preferably 80 mass% or more, more preferably 90 mass% or more, still more preferably 95 mass% or more, particularly preferably 100 mass%.

The esterified compound (A) may include, as a constituent component, a component other than the triol (a1). Examples of the component include diols such as ethylene glycol, propylene glycol, 1,4-butanediol, 1,5-pentanediol, 3-methyl-1,5-pentanediol, and 1,6-hexanediol; tetraols such as pentaerythritol, sorbitan, and mannitan; and hexaols such as sorbitol and mannitol.

### Fatty acid (a2) having 4 to 12 carbon atoms

The fatty acid (a2) having 4 to 12 carbon atoms is a hydrocarbon compound having 3 to 11 carbon atoms in which a single hydrogen atom is substituted with a carboxy group. The fatty acid (a2) having 4 to 12 carbon atoms is not particularly limited. Examples of the fatty acid (a2) having 4 to 12 carbon atoms include saturated fatty acids such as butanoic acid, pentanoic acid, hexanoic acid, heptanoic acid, octanoic acid, nonanoic acid, decanoic acid, and dodecanoic acid; and unsaturated fatty acids such as butenoic acid, pentenoic acid, hexenoic acid, heptenoic acid, octenoic acid, nonenoic acid, decenoic acid, and dodecenoic acid. Such fatty acids (a2) having 4 to 12 carbon atoms may be used in combination. In particular, the fatty acid (a2) having 4 to 12 carbon atoms is, from the viewpoint of providing a polyurethane resin having higher heat resistance, preferably a saturated fatty acid. The fatty acid (a2) having 4 to 12 carbon atoms is, from the viewpoint of exhibiting a lower resin viscosity during shaping, a fatty acid having 4 to 12 carbon atoms, preferably a saturated fatty acid having 4 to 12 carbon atoms.

The content of the fatty acid (a2) having 4 to 12 carbon atoms is not particularly limited. For example, the content of the fatty acid (a2) having 4 to 12 carbon atoms of the carboxylic acid contained in the constituent components of the esterified compound (A) is, from the viewpoint of exhibiting a lower viscosity during shaping, preferably 90 mass% or more, more preferably 95 mass% or more, still more preferably 98 mass% or more, particularly preferably 100 mass%.

### Other constituent component

The esterified compound (A) may include, as a constituent component, a monocarboxylic acid (a3) other than the fatty acid (a2) having 4 to 12 carbon atoms. The monocarboxylic acid (a3) is not particularly limited. Examples of the monocarboxylic acid (a3) include hydroxyl group-containing fatty acids such as ricinoleic acid.

The monocarboxylic acid (a3) is not particularly limited in terms of content proportion. The content proportion of the monocarboxylic acid (a3) relative to 1 mole of the fatty acid (a2) having 4 to 12 carbon atoms is, from the viewpoint of exhibiting a lower viscosity during shaping and providing a polyurethane resin having a higher glass transition temperature, preferably 0.2 moles or less, more preferably 0.1 moles or less, still more preferably 0.05 moles or less.

Hereafter, the esterified compound (A) as a whole will be further described. The esterified compound (A) has an average esterification degree of 0.5 to 2.5. In this embodiment, the average esterification degree is the average number of ester groups contained in a single molecule. When the average esterification degree satisfies such a range, the polyol composition for a polyurethane resin tends to exhibit a low viscosity during shaping and tends to provide a polyurethane resin having a higher glass transition temperature. The average esterification degree is at least 0.5 or more, preferably 0.8 or more, more preferably 1.0 or more. The average esterification degree is at least 2.5 or less, preferably 2.0 or less, more preferably 1.8.

The esterified compound (A) content of the polyol composition for a polyurethane resin is, from the viewpoint of exhibiting a lower viscosity during shaping, 80 mass% or more. The esterified compound (A) content of the polyol composition for a polyurethane resin is, from the viewpoint of advantageously providing a polyurethane resin having a higher glass transition temperature, preferably 100 mass% or less, more preferably 95 mass% or less, still more preferably 90 mass% or less.

The method for producing the esterified compound (A) according to this embodiment is not particularly limited. For example, the esterified compound (A) can be obtained by a publicly known method such as reacting the triol (a1), the fatty acid (a2) having 4 to 12 carbon atoms, optionally the monocarboxylic acid (a3) other than the fatty acid (a2) having 4 to 12 carbon atoms, and a reaction solvent in the presence of an esterification catalyst and at 100°C to 180°C while water generated as a by-product is removed. Alternatively, the esterified compound (A) of this embodiment may be a commercially available product.

### (Polyol (B))

Hereafter, the polyol composition for a polyurethane resin, as a whole, will be further described. The polyol composition for a polyurethane resin according to this embodiment preferably further includes the polyol (B) having a molecular weight of 500 or less.

The polyol (B) is not particularly limited. Examples of the polyol (B) include diols such as 1,4-butanediol, 1,6-hexanediol, 3-methyl-1,5-pentanediol, bisphenol A, bisphenol S, and bisphenol F; triols such as glycerin and trimethylolpropane; tetraols such as pentaerythritol, sorbitan, and mannitan; and hexaols such as sorbitol and mannitol. Alternatively, the polyol (B) may be a polyether polyol in which an alkylene oxide (for example, ethylene oxide, propylene oxide, or butylene oxide) is added to such a polyol. Such polyols (B) may be used in combination. In particular, the polyol (B) is, from the viewpoint of exhibiting a low viscosity during shaping and providing a polyurethane resin having a higher glass transition temperature, preferably a polyol having a molecular weight of 300 or less, more preferably a polyol having a molecular weight of 200 or less. The polyol (B) is, from the viewpoint of exhibiting a low viscosity during shaping and providing a polyurethane resin having a higher glass transition temperature, preferably a triol, more preferably trimethylolpropane.

The polyol (B) content is not particularly limited. For example, the polyol (B) content relative to 100 parts by mass of the esterified compound (A) is preferably 0.1 mass% or more, more preferably 1 mass% or more, still more preferably 5 mass% or more. The polyol (B) content relative to 100 parts by mass of the esterified compound (A) is preferably 60 mass% or less, more preferably 50 mass% or less, still more preferably 40 mass% or less. When the polyol (B) content satisfies such a range, the resultant polyol composition for a polyurethane resin tends to exhibit a lower viscosity during shaping and tends to provide a polyurethane resin having a higher glass transition temperature.

### (Optional component)

The polyol composition for a polyurethane resin according to this embodiment may include, in addition to the above-described esterified compound (A) and polyol (B) having a molecular weight of 500 or less, another polyol. Examples of the other polyol include polyether polyol, polyester polyol, polybutadiene polyol, polycarbonate polyol, polyisoprene polyol, hydrogenated polybutadiene polyol, and hydrogenated polyisoprene polyol. When the other polyol is included, the other polyol content is not particularly limited. For example, the other polyol content of the polyol composition for a polyurethane resin is 1 to 30 mass% from the viewpoint of exhibiting a low viscosity during shaping and providing a polyurethane resin having a high glass transition temperature.

The polyol composition for a polyurethane resin according to this embodiment may optionally include various additives such as a plasticizer, a flame retardant, a catalyst, an antioxidant, a moisture absorbent, a fungicide,
a silane coupling agent, a defoaming agent, a surface control agent, and an internal release agent. Examples of the silane coupling agent include alkoxysilanes, vinyl group-containing silane coupling agents, epoxy group-containing silane coupling agents, methacryloyl group-containing silane coupling agents, and acryloyl group-containing silane coupling agents.

### <Polyurethane resin-forming composition>

A polyurethane resin-forming composition according to an embodiment of the present invention includes the above-described polyol composition for a polyurethane resin and an isocyanate group-containing compound (C).

### (Isocyanate group-containing compound (C))

The isocyanate group-containing compound (C) is not particularly limited as long as it is a compound intramolecularly having an isocyanate group. Examples of the isocyanate group-containing compound (C) include aliphatic polyisocyanate compounds, alicyclic polyisocyanate compounds, aromatic polyisocyanate compounds, and aromatic and aliphatic polyisocyanate compounds. Such isocyanate group-containing compounds (C) may be used in combination.

The aliphatic polyisocyanate compounds are not particularly limited. Examples of the aliphatic polyisocyanate compounds include tetramethylene diisocyanate, dodecamethylene diisocyanate, hexamethylene diisocyanate (HDI), 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate. Of these, such an aliphatic polyisocyanate compound is preferably hexamethylene diisocyanate (HDI) from the viewpoint of exhibiting a lower viscosity during shaping, achieving higher productivity, and providing a polyurethane resin having a higher tensile strength and a higher elongation at break.

The alicyclic polyisocyanate compounds are not particularly limited. Examples of the alicyclic polyisocyanate compounds include isophorone diisocyanate, hydrogenated xylylene diisocyanate, 4,4'-dicyclohexylmethane diisocyanate, 1,4-cyclohexane diisocyanate, methylcyclohexylene diisocyanate, and 1,3-bis(isocyanatomethyl)cyclohexane. Of these, such an alicyclic polyisocyanate compound is preferably isophorone diisocyanate.

The aromatic polyisocyanate compounds are not particularly limited. Examples of the aromatic polyisocyanate compounds include tolylene diisocyanate (TDI), 2,2'-diphenylmethane diisocyanate, 2,4'-diphenylmethane diisocyanate, 4,4'-diphenylmethane diisocyanate (MDI), polymethylene polyphenyl polyisocyanate (polymeric MDI), 4,4'-dibenzyl diisocyanate, 1,5-naphthylene diisocyanate, xylylene diisocyanate, 1,3-phenylene diisocyanate, and 1,4-phenylene diisocyanate. Of these, such an aromatic polyisocyanate compound is, from the viewpoint of providing a polyurethane resin having a higher tensile strength, preferably polymethylene polyphenyl polyisocyanate (polymeric MDI).

The aromatic and aliphatic polyisocyanate compounds are not particularly limited. Examples of the aromatic and aliphatic polyisocyanate compounds include dialkyldiphenylmethane diisocyanate, tetraalkyldiphenylmethane diisocyanate, and α,α,α,α-tetramethylxylylene diisocyanate. Of these, such an aromatic and aliphatic polyisocyanate compound is, from the viewpoint of providing a polyurethane resin having a higher elongation at break, preferably α,α,α,α-tetramethylxylylene diisocyanate.

Other examples of the polyisocyanate compounds include modified compounds such as isocyanate group-terminated urethane prepolymer modified compounds provided by a reaction between an isocyanate group-containing compound and a hydroxyl group-containing compound, carbodiimide modified compounds, isocyanurate modified compounds, allophanate modified compounds, and biuret modified compounds. These may be used in combination.

The polyisocyanate compounds are, from the viewpoint of exhibiting a lower viscosity during shaping and providing a polyurethane resin having a higher glass transition temperature, preferably aliphatic polyisocyanate compounds, aromatic polyisocyanate compounds, and the above-described modified compounds of these, more preferably MDI, HDI, and modified compounds of these, still more preferably isocyanurate modified compounds of MDI and HDI.

Hereafter, the polyurethane resin-forming composition as a whole will be further described. The polyurethane resin-forming composition according to this embodiment preferably has a molar ratio of isocyanate groups to hydroxyl groups (NCO/OH) of 0.5 or more, more preferably 0.6 or more, still more preferably 0.8 or more. The polyurethane resin-forming composition preferably has a molar ratio of isocyanate groups to hydroxyl groups (NCO/OH) of 1.5 or less, more preferably 1.3 or less, still more preferably 1.2 or less. When the molar ratio of isocyanate groups to hydroxyl groups (NCO/OH) satisfies such a range, the polyurethane resin-forming composition is less likely to cause curing failure.

The polyurethane resin-forming composition according to this embodiment is not particularly limited in terms of mixing viscosity in an environment at 25°C. For example, the viscosity is preferably 1000 mPa·s or less, more preferably 800 mPa·s or less, still more preferably 600 mPa·s or less, particularly preferably 300 mPa·s or less. The mixing viscosity can be measured by a method described later in EXAMPLES.

The polyurethane resin-forming composition according to this embodiment is not particularly limited in terms of pot life (the length of time taken to reach 1000 mPa·s) in an environment at 25°C. For example, the pot life (the length of time taken to reach 1000 mPa·s) is preferably 10 minutes or more, more preferably 20 minutes or more. The pot life (the length of time taken to reach 1000 mPa·s) is, from the viewpoint of achieving higher productivity, preferably 60 minutes or less, more preferably 50 minutes or less. The pot life (the length of time taken to reach 1000 mPa·s) can be measured by a method described later in EXAMPLES.

A polyurethane resin-forming composition according to the present invention is not particularly limited in terms of pot life (the length of time taken to reach 10000 mPa·s) in an environment at 25°C. For example, the pot life (the length of time taken to reach 10000 mPa·s) is preferably 20 minutes or more, more preferably 30 minutes or more, still more preferably 40 minutes or more. The pot life (the length of time taken to reach 10000 mPa·s) is, from the viewpoint of achieving higher productivity, preferably 80 minutes or less, more preferably 60 minutes or less. The pot life (the length of time taken to reach 10000 mPa·s) can be measured by a method described later in EXAMPLES.

### <Composite material>

A composite material according to an embodiment of the present invention includes a cured product of the above-described polyurethane resin-forming composition and a reinforcing fiber.

The cured product of the polyurethane resin-forming composition is not particularly limited in terms of glass transition temperature. For example, the glass transition temperature is preferably 80°C or more, more preferably 90°C or more, still more preferably 100°C or more, particularly preferably 110°C or more. The glass transition temperature can be measured by a method described later in EXAMPLES.

### (Reinforcing fiber)

The reinforcing fiber is not particularly limited. Examples of the reinforcing fiber include carbon fiber, glass fiber, aramid fiber, alumina fiber, silicon carbide fiber, boron fiber, metallic fiber, natural fiber, and mineral fiber. Such reinforcing fibers may be used in combination. In particular, preferred reinforcing fibers are, from the viewpoint of having high strength and high rigidity and being lightweight, PAN-based, pitch-based, or rayon-based carbon fiber, for example. The reinforcing fiber is, from the viewpoint of achieving higher profitability, preferably glass fiber, more preferably includes carbon fiber and glass fiber. The reinforcing fiber includes, from the viewpoint of providing a shaped article having improved properties such as shock absorbency, preferably aramid fiber, more preferably includes carbon fiber and aramid fiber. The reinforcing fiber may be, from the viewpoint of providing a shaped article having improved electroconductivity, a reinforcing fiber covered with a metal such as nickel.

In the carbon fiber, a surface oxygen concentration ratio (O/C), which is measured by X-ray photoelectron spectroscopy (XPS) and is a ratio of the number of oxygen (O) atoms to the number of carbon (C) atoms, is preferably 0.05 or more, more preferably 0.08 or more. The surface oxygen concentration ratio (O/C) is preferably 0.50 or less, more preferably 0.40 or less. When the surface oxygen concentration ratio (O/C) satisfies such a range, the obtained carbon fiber tends to exhibit high adhesion to a sizing agent and tends to have excellent dynamic characteristics.

The reinforcing fiber is not particularly limited in terms of average fiber diameter. For example, the average fiber diameter is, from the viewpoint of the dynamic characteristics and surface appearance of the resultant composite material, preferably 1 µm or more, more preferably 3 µm or more. The average fiber diameter is preferably 20 µm or less, more preferably 15 µm or less.

The reinforcing fiber may be a reinforcing fiber bundle composed of a plurality of single yarns. In this case, the number of single yarns may be 100 to 350000, for example.

The reinforcing fiber is not particularly limited in terms of number-average fiber length. For example, the number-average fiber length is preferably 0.1 mm or more. The number-average fiber length is preferably 50 mm or less, more preferably 20 mm or less. The method of measuring the number-average fiber length is not particularly limited. For example, the number-average fiber length can be measured by the following method: the resin components contained in the composite material are removed by a process such as a dissolution process or an incineration process; the residual reinforcing fiber is isolated by filtration, and is subsequently observed with a microscope.

In order to improve the adhesion of the reinforcing fiber in the composite material, a compound having, in a single molecule, two or more functional groups of at least one species selected from the group consisting of a carboxyl group, a hydroxyl group, an amino group, and an epoxy group preferably adheres to the reinforcing fiber.

Examples of the compound include polyfunctional epoxy resins, acrylic acid-based polymers, polyhydric alcohols, and polyethyleneimines.

Examples of the polyfunctional epoxy resins include tri- or higher functional aliphatic epoxy resins and phenol-novolac epoxy resins. Examples of the tri- or higher functional aliphatic epoxy resins include polyglycidyl ethers of aliphatic polyhydric alcohols such as glycerol triglycidyl ether, diglycerol polyglycidyl ether, polyglycerol polyglycidyl ether, sorbitol polyglycidyl ether, arabitol polyglycidyl ether, trimethylolpropane triglycidyl ether, and pentaerythritol polyglycidyl ether. The acrylic acid-based polymers mean polymers synthesized from acrylic acid, methacrylic acid, or maleic acid and having three or more carboxyl groups in a single molecule; examples include polyacrylic acid, copolymers of acrylic acid and methacrylic acid, copolymers of acrylic acid and maleic acid, and mixtures of two or more of the foregoing. Examples of the polyhydric alcohols include polyvinyl alcohol, glycerol, diglycerol, polyglycerol, sorbitol, arabitol, trimethylolpropane, and pentaerythritol. The polyethyleneimines are polyamines obtained by ring-opening polymerization of ethyleneimine and having a branched structure of primary, secondary, or tertiary amino groups; examples include a polyethyleneimine including more amino groups in a single molecule.

In order to improve adhesion of the reinforcing fiber in the composite material, a polyurethane resin preferably adheres to the reinforcing fiber. The polyurethane resin is preferably provided by applying an aqueous dispersion of polyurethane resin, and drying the aqueous dispersion.

When the above-described compound is included, the content of the compound relative to 100 parts by mass of the reinforcing fiber is, from the viewpoint of efficiently improving the strength of the composite material, preferably 0.01 parts by mass or more, more preferably 0.1 parts by mass or more. The content of the compound relative to 100 parts by mass of the reinforcing fiber is preferably 5 parts by mass or less, more preferably 2 parts by mass or less.

The method of causing the compound to adhere to the reinforcing fiber is not particularly limited. For example, a roller may be used to cause the compound to adhere, as a sizing agent, to the reinforcing fiber.

Hereafter, the composite material as a whole will be further described. In the composite material, the mixing proportions of the cured product of the polyurethane resin-forming composition and the reinforcing fiber are not particularly limited. For example, the amount of reinforcing fiber mixed relative to 100 parts by mass of the cured product of the polyurethane resin-forming composition is, from the viewpoint of providing higher strength or higher heat resistance, preferably 20 parts by mass or more, more preferably 40 parts by mass or more. The amount of reinforcing fiber mixed relative to 100 parts by mass of the cured product of the polyurethane resin-forming composition is, from the viewpoint of providing better mechanical properties such as strength, preferably 180 parts by mass or less, more preferably 150 parts by mass or less.

The method for producing the composite material according to this embodiment is not particularly limited. For example, the composite material can be produced by a method of applying and impregnating the polyurethane resin-forming composition to the reinforcing fiber, and subsequently curing the polyurethane resin-forming composition. The step of applying and impregnating the polyurethane resin-forming composition may be performed by a publicly known process (for example, a brush or a roller). The step of curing the polyurethane resin-forming composition may be performed, as needed, in an environment under heating (for example, at 60°C to 180°C) or in an environment under a reduced pressure (for example, at 5 kPa or less). In this case, the composite material according to this embodiment employs the above-described polyol composition for a polyurethane resin, which provides improved usability during shaping.

The composite material according to this embodiment is suitable as housings of electronic devices, and is suitably used for computers, televisions, cameras, and audio players, for example. The composite material is also suitably used for electric and electronic components, and is suitably used for connectors, LED lamps, sockets, optical pickup devices, terminal boards, printed boards, speakers, small motors, magnetic heads, power modules, dynamos, electric motors, transformers, converters, voltage regulators, rectifiers, and inverters, for example. The composite material is also suitably used for automotive parts and vehicle-related parts, for example, and are suitably used for safety belt parts, instrument panels, console boxes, pillars, roof rails, fenders, bumpers, door panels, roof panels, hood panels, trunk lids, door mirror stays, spoilers, hood louvers, wheel covers, wheel caps, garnish, intake manifolds, fuel pumps, engine coolant joints, windshield washer nozzles, wipers, battery-related parts, wire harness connectors, lamp housings, lamp reflectors, and lamp sockets, for example. In addition, the composite material is suitable as building materials, and is suitably used for, in constructions, walls, roofs, ceiling-related components, window material-related components, insulator-related components, floor material-related components, seismic isolation or damping material-related components, and lifeline-related components, for example. In addition, the composite material is suitable as sports goods, and is suitably used for golf-related goods such as shafts of golf clubs and golf balls; sports racket-related goods such as tennis rackets and badminton rackets; sports body protector goods for, for example, American football, baseball, or softball, such as masks, helmets, chest protectors, elbow guards, and knee guards; fishing-related goods such as fishing rods, reels, and lures; and winter sports-related goods for skiing and snowboarding, for example.

### EXAMPLES

Hereinafter, the present invention will be described more specifically with reference to Examples. The present invention is not limited to these Examples at all.

The following are the method for producing the esterified compound (A) and starting materials used.

### (Production Example 1)

A reaction vessel equipped with a thermometer, a reflux condenser, and a stirring device was charged with 1000 g of trimethylolpropane, 1210 g of octanoic acid, and 0.5 g of tetrabutyl titanate. In order to provide a column top temperature of 100°C, the reaction temperature was set at 160°C. While water generated as a by-product was removed out of the reaction system, the reaction was caused until the acid value became 0.5 mgKOH/g or less. Subsequently, dehydration was performed at a reduced pressure at 100°C for 1 hour, to provide an esterified compound 1 (average hydroxyl value: 363 mgKOH/g, average esterification degree: 1.1) containing 5 mass% of unreacted trimethylolpropane.

### (Production Example 2)

The same procedures as in Production Example 1 were performed except that the amount of octanoic acid was changed to 1684 g, to provide an esterified compound 2 (average hydroxyl value: 239 mgKOH/g, average esterification degree: 1.5) containing 0.8 mass% of unreacted trimethylolpropane.

### (Production Example 3)

The same procedures as in Production Example 1 were performed except that the amount of octanoic acid was changed to 1283 g, to provide an esterified compound 3 (average hydroxyl value: 171 mgKOH/g, average esterification degree: 1.8) containing 0.3 mass% of unreacted trimethylolpropane.

### (Production Example 4)

The same procedures as in Production Example 1 were performed except that the octanoic acid was replaced by 1358 g of hexanoic acid, to provide an esterified compound 4 (average hydroxyl value: 272 mgKOH/g, average esterification degree: 1.5) containing 0.8 mass% of unreacted trimethylolpropane.

### (Production Example 5)

The same procedures as in Production Example 1 were performed except that the octanoic acid was replaced by 2338 g of dodecanoic acid, to provide an esterified compound 5 (average hydroxyl value: 193 mgKOH/g, average esterification degree: 1.5) containing 0.8 mass% of unreacted trimethylolpropane.

### (Production Example 6)

The same procedures as in Production Example 1 were performed except that the octanoic acid was replaced by 2193 g of hexadecanoic acid, to provide an esterified compound 6 (average hydroxyl value: 256 mgKOH/g, average esterification degree: 1.5) containing 5 mass% of unreacted trimethylolpropane.

### (Production Example 7)

The same procedures as in Production Example 1 were performed except that the trimethylolpropane was replaced by 515 g of glycerin, to provide an esterified compound 7 (average hydroxyl value: 273 mgKOH/g, average esterification degree: 1.5) containing 0.9 mass% of unreacted glycerin.

### Starting materials used

B-1: trimethylolpropane
B-2: glycerin
B-3: propylene oxide adduct of bisphenol A (ADEKA POLYETHER BPX-11, average hydroxyl value: 310 mgKOH/g)
B-4: 3-methyl-1,5-pentanediol
C-1: polymeric MDI (FOAM LIGHT 500B, manufactured by BASF INOAC Polyurethanes Ltd.)
C-2: isocyanurate-modified HDI (TAKENATE D-170HN, manufactured by Mitsui Chemicals, Inc.)

### (Examples 1 to 5 and 7 to 12, and Reference Example 6)

In accordance with starting materials and mixing proportions described in Table 1, components other than the isocyanate group-containing compound (C) were mixed, to provide polyol compositions for polyurethane resins. In Table 1, the amounts of esterified compounds of Production Examples 1 to 7 are described so as to exclude the amounts of unreacted trimethylolpropane or glycerin; the amounts of trimethylolpropane or glycerin are described so as to include unreacted compounds contained in Production Examples 1 to 7. The obtained polyol compositions for polyurethane resins were set at 25°C, and mixed with the isocyanate group-containing compound (C) set at 25°C, in proportions described in Table 1 for 1 minute.

### [Table 1]

**Table 1**

| | | Examples | | | | | | | | | | |
|---|---|---|---|---|---|---|---|---|---|---|---|---|
| | | 1 | 2 | 3 | 4 | 5 | 6* | 7 | 8 | 10 | 11 | 12 |
| Starting materials | Esterified compound 1 | | - | - | - | | | | | | | |
| | Esterified compound 2 | | | | | | | | 90 | 80 | 80 | 85 |
| | Esterified compound 3 | - | - | 85 | - | | | | | | | - |
| | Esterified compound 4 | - | - | | 85 | | | | | | | - |
| | Esterified compound 5 | - | - | | - | 85 | | | | | | - |
| | Esterified compound 6 | - | - | | - | | 85 | | | | | - |
| | Esterified compound 7 | | | | | | | 85 | | | | |
| | B-1 | | 15 | 15 | 15 | 15 | 15 | - | - 10 | 10 | 10 | 15 |
| | B-2 | - | - | | - | - | | 15 | - | | - | - |
| | B-3 | - | - | | - | - | | - | - | 10 | - | - |
| | B-4 | - | - | | - | - | | - | - | | 10 | - |
| | Zeolite | 1 | 1 | 1 | 1 | 1 | 1 | 1 | 1 | 1 | 1 | 1 |
| | C-1 | 120 | 94 | 80 | 101 | 85 | 97 | 101 | 82 | 84 | 99 | - |
| | C-2 | | - | | | | | - | | | - | 132 |
| Evaluations | Mixing viscosity (mPa·s) | 240 170 | 170 | 130 | 130 140 | 550 | 760 | 155 | 110 | 390 | 150 | 450 |
| | Glass transition temperature (°C) | 137 | 116 | 100 | 110 | 100 | 95 | 104 | 95 | 128 | 112 | 145 |
| | Tensile strength (MPa) | 62 | 61 | 59 | 59 | 48 | 39 | 62 | 46 | 68 | 62 | 46 |
| | Elongation at break (%) | 2.2 | 2.5 | 2.8 | 2.1 | 4.5 | 5.2 | 2.1 | 3.9 | 1.5 | 2.3 | 2.9 |
| | Pot life 1 | B | A | A | A | A | B | A | A | B | B | B |
| | Pot life 2 | C | B | A | B | A | A | B | A | B | B | A |

| | | | | | | | | | | | | |
|---|---|---|---|---|---|---|---|---|---|---|---|---|
| *Reference Examples useful to understand the invention | | | | | | | | | | | | |

The polyol compositions for polyurethane resins and the polyurethane resin-forming compositions obtained in Examples 1 to 5 and 7 to 12, and Reference Example 6 were measured by the following evaluation methods in terms of mixing viscosity, glass transition temperature, tensile strength, elongation at break, pot life 1, and pot life 2. The results are described in Table 1.

### <Evaluation methods>

### (1) Mixing viscosity (mPa·s)

The above-described mixtures were each left to stand in an environment at 25°C, and measured in terms of viscosity after 3 minutes elapsed from the start of mixing. The viscosity was measured in accordance with JIS K-7117-1 and with a BM-type viscometer (manufactured by Toki Sangyo Co., Ltd.).

### (2) Glass transition temperature (°C)

The above-described mixtures were each applied so as to form a film having a thickness of 1 mm. This was treated at 80°C for 1 hour and at 150°C for 16 hours, to obtain a polyurethane resin sheet. From the obtained sheet, a 5 mm × 2 cm test piece was cut out, and measured in terms of glass transition temperature, in accordance with JIS K-7244-4, and with a Rheogel E-4000, manufactured by UBM.

### (3) Tensile strength (MPa) and elongation at break (%)

The same method as in the glass transition temperature was performed to obtain a polyurethane resin sheet having a film thickness of 1 mm.

From the obtained sheet, a 5 mm × 4 cm test piece was cut out, and measured in terms of tensile strength (MPa) and elongation at break (%), in accordance with JIS A6021-2011, and with a digital universal testing machine (Instron 5581) manufactured by Instron Japan Co., Ltd.

### (4) Pot life 1 and pot life 2

The above-described mixtures were each left to stand in an environment at 25°C, and measured in terms of viscosity every 2 minutes after 4 minutes elapsed from the start of mixing; the time at which the viscosity exceeds 1000 mPa·s was determined as pot life 1, and the time at which the viscosity exceeds 10000 mPa·s was determined as pot life 2. The viscosity was measured in accordance with JIS Z8803 and with a BM-type viscometer (manufactured by Toki Sangyo Co., Ltd.). The following are grading systems.

### (Grading system of pot life 1)

A: 20 minutes or more and 60 minutes or less
B: 10 minutes or more and less than 20 minutes
C: less than 10 minutes

### (Grading system of pot life 2)

A: 40 minutes or more and 80 minutes or less
B: 30 minutes or more and less than 40 minutes
C: 20 minutes or more and less than 30 minutes
D: 10 minutes or more and less than 20 minutes
E: less than 10 minutes

As described in Table 1, the mixtures of polyol compositions for polyurethane resins according to the present invention have been demonstrated to exhibit such low mixing viscosities that they are highly usable. In addition, these mixtures have such high glass transition temperatures that they are seemingly suitable for various applications. Furthermore, the obtained sheets have such high tensile strengths and elongations at break that they are seemingly suitable for various applications. The results of pot life 1 and pot life 2 have demonstrated that polyol compositions for polyurethane resins according to the present invention have such long pot lives that they are highly usable.

## Claims

1. A polyol composition for a polyurethane resin, comprising an esterified compound (A) having an average esterification degree of 0.5 to 2.5, wherein the average esterification degree is the average number of ester groups contained in a single molecule,
wherein the esterified compound (A) includes, as constituent components, a triol (a1) and a fatty acid (a2) having 4 to 12 carbon atoms and wherein
the esterified compound (A) content of the polyol composition for a polyurethane resin is 80 mass% or more.

2. The polyol composition for a polyurethane resin according to Claim 1, further comprising a polyol (B) having a molecular weight of 500 or less.

3. The polyol composition for a polyurethane resin according to Claim 2, wherein the polyol (B) is a triol.

4. A polyurethane resin-forming composition comprising the polyol composition for a polyurethane resin according to any one of Claims 1 to 3 and an isocyanate group-containing compound (C).

5. A composite material comprising a cured product of the polyurethane resin-forming composition according to Claim 4 and a reinforcing fiber.

## Patentansprüche

1. Polyolzusammensetzung für ein Polyurethanharz, umfassend eine veresterte Verbindung (A) mit einem durchschnittlichen Veresterungsgrad von 0,5 bis 2,5, wobei der durchschnittliche Veresterungsgrad die durchschnittliche Anzahl der in einem einzelnen Molekül enthaltenen Estergruppen ist,
wobei die veresterte Verbindung (A) als konstituierende Komponenten ein Triol (a1) und eine Fettsäure (a2) mit 4 bis 12 Kohlenstoffatomen enthält und wobei
der Gehalt der veresterten Verbindung (A) in der Polyolzusammensetzung für ein Polyurethanharz 80 Masse-% oder mehr beträgt.

2. Polyolzusammensetzung für ein Polyurethanharz nach Anspruch 1, ferner umfassend ein Polyol (B) mit einem Molekulargewicht von 500 oder weniger.

3. Polyolzusammensetzung für ein Polyurethanharz nach Anspruch 2, wobei das Polyol (B) ein Triol ist.

4. Polyurethanharz-bildende Zusammensetzung, umfassend die Polyolzusammensetzung für ein Polyurethanharz nach einem der Ansprüche 1 bis 3 und eine Isocyanatgruppen-enthaltende Verbindung (C).

5. Verbundmaterial, umfassend ein gehärtetes Produkt der Polyurethanharzbildenden Zusammensetzung nach Anspruch 4 und eine Verstärkungsfaser.

## Revendications

1. Composition de polyol pour une résine de polyuréthane, comprenant un composé estérifié (A) ayant un degré d'estérification moyen de 0,5 à 2,5, dans laquelle le degré d'estérification moyen est le nombre moyen de groupes esters contenus par molécule,
dans laquelle le composé estérifié (A) comprend, en tant que composants constitutifs, un triol (a1) et un acide gras (a2) ayant 4 à 12 atomes de carbone,
et dans laquelle la teneur en le composant estérifié (A) de la composition de polyol pour une résine de polyuréthane est de 80 % en masse ou plus.

2. Composition de polyol pour une résine de polyuréthane selon la revendication 1, comprenant en outre un polyol (B) ayant une masse moléculaire de 500 ou moins.

3. Composition de polyol pour une résine de polyuréthane selon la revendication 2, dans laquelle le polyol (B) est un triol.

4. Composition formant une résine de polyuréthane comprenant la composition de polyol pour une résine de polyuréthane selon l'une quelconque des revendications 1 à 3 et un composé contenant un groupe isocyanate (C).

5. Matériau composite comprenant un produit durci de la composition formant une résine de polyuréthane selon la revendication 4 et des fibres de renforcement.
